# EUROPÄISCHE PATENTANMELDUNG

(11) **EP 4 843 538 A1**
(43) Veröffentlichungstag der Anmeldung: **25.11.2026**
(21) Anmeldenummer: 25178098.7
(22) Anmeldetag: 22.05.2025
(51) Int. Cl.: A61F 2/30, A61F 2/34, A61F 2/46

(54) **GIESSFORM FÜR ACETABULUM-SPACER MIT ZENTRIERUNG**

(71) Anmelder: Heraeus Medical GmbH, 61273 Wehrheim (DE)
(72) Erfinder: Dr. Vogt, Sebastian, 61273 Wehrheim (DE); Dr. Kluge, Thomas, 61273 Wehrheim (DE); Dr. Nassut, Roman, 61273 Wehrheim (DE)
(74) Vertreter: Heraeus IP

(57) **Zusammenfassung**

Die Erfindung betrifft eine Gießform zur Herstellung eines Acetabulum-Spacers, aufweisend ein Bodenteil (**100**) mit einem Rand (**101**), der das Bodenteil (**100**) nach außen begrenzt, einer gewölbten, kreisförmigen Mulde (**102**) und einer Sicke (**103**), die umlaufend um die Mulde (**102**) angeordnet ist, wobei die Sicke (**103**) zur Aufnahme von überschüssigem Knochenzement ausgebildet und eingerichtet ist,
und ein Deckelteil (**200**) mit einer Vielzahl von Zentrierelementen (**201**), welche ausgebildet und eingerichtet sind, in den Rand (**101**) einzugreifen, und das Deckelteil (**200**) zentriert an dem Bodenteil (**100**) auszurichten.

## Beschreibung

### GEBIET DER ERFINDUNG

Die vorliegende Erfindung betrifft das Gebiet der Medizintechnik, insbesondere Vorrichtungen zur Herstellung medizinischer Implantate, und die damit herstellbaren medizinischen Implantate selbst. Weiterhin betrifft die Erfindung ein Herstellungsverfahren, in dem die erfindungsgemäßen Vorrichtungen zur Herstellung medizinischer Implantate verwendet werden.

### TECHNISCHER HINTERGRUND

Spacer aus Polymethylmethacrylat-Knochenzement, die ein oder mehrere Antibiotika enthalten können, werden mit Erfolg beim zweizeitigen septischen Wechsel von infizierten Gelenkendoprothesen verwendet. Die Spacer werden dazu nach der Entfernung der infizierten Gelenkendoprothese und nachfolgendem Debridement als temporäre Implantate für die Interimszeit bis zur Implantation der Revisionsgelenkendoprothese eingesetzt. In der Interimszeit können aus den Spacern ein oder mehrere Antibiotika freigesetzt werden, die zum Beruhigen der Infektion beitragen können. Die Spacer werden zum "Deadspace-Management" eingesetzt, d. h. der Behandlung von chirurgisch- oder infektionsbedingten Hohlräumen, und können eine Atrophie des Bandapparates und der Muskulatur verhindern.

Bei der zweizeitigen septischen Revision von Hüftgelenkendoprothesen werden der Prothesenschaft mit dem Prothesenkopf und die Gelenkpfanne in einer ersten OP entfernt. Als Hüftgelenkspacer finden in der Interimsphase dabei häufig aus Polymethlymethacrylat (PMMA) gefertigte Schäfte mit einem kugelförmigen oder pilzförmigen Kopf Verwendung. Die Köpfe besitzen einen so großen Durchmesser, dass diese direkt in das Acetabulum (Hüftgelenkpfanne) auf das Knochengewebe eingesetzt werden können. Problematisch ist jedoch, dass die Spacerköpfe während der Artikulation auf der Knochenoberfläche des Acetabulums reiben können.

Alternativ dazu wurden sog. Acetabulum-Cup-Spacer entwickelt, welche die Gelenkpfanne nachbilden. Bei diesen handelt es sich um schalenförmige Spacer mit einer konkaven Fläche zur Aufnahme des Spacerkopfs und einer konvexen Rückseite, die mit Polymethylmethacrylat-Knochenzementteig im Acetabulum verankert werden können.

In EP3761909EPB1 wird ein Acetabulum-Cup-Spacer vorgeschlagen, der halbkugelförmig ausgebildet ist. Auf der Außenseite erstrecken sich vom Rand des Spacers Aussparungen oder auch Stege, die zum Zentrum der der Halbkugel ausgerichtet sind.

In US12127944B2 wird eine zweiteilige Gießform vorgeschlagen, die aus einem konkaven und einem konvexen Teil bestehen. Der konkave Teil der Gießform wird mit dem konvexen Teil durch Verschraubung verbunden. Der konvexe Teil enthält eine Öffnung zum Einpressen von Polymethylmethacrylat-Knochenzement. Weiterhin sind röhrenförmige Entlüftungen am Rand des konvexen Teils angeordnet. An der Trennung der beiden Teile kann sich ein Grat am gegossenen Spacer ausbilden. Grate können sehr scharfkantig sein und können nur aufwendig mechanisch entfernt werden, insbesondere wenn diese eine Dicke von etwa 0,3 mm oder mehr haben. Solche größeren Grate können entweder mit Hilfe von Zangen abgebrochen oder mit Fräsern abgefräst werden, was für den medizinischen Anwender mühsam ist und eine gewünschte Formung des Spacers erschwert.

### KURZE BESCHREIBUNG DER ERFINDUNG

Eine Aufgabe der Erfindung ist die Bereitstellung einer Gießform, welche insbesondere für die intraoperative Herstellung von Acetabulum-Cup-Spacern aus Polymethylmethacrylat-Knochenzement verwendet werden kann.

Eine Aufgabe der Erfindung ist insbesondere die Bereitstellung einer Gießform, die leicht von einem medizinischen Anwender mit Knochenzement befüllt werden kann. Beispielsweise lassen sich die erfindungsgemäßen Gießformen ohne die Verwendung einer Austragsvorrichtung mit Knochenzement befüllen. Ein medizinischer Anwender kann beispielsweise manuell gemischten Knochenzement mithilfe eines Spatels in die Gießform streichen.

Eine weitere Aufgabe der Erfindung ist ein einfaches Zusammenbauen der Gießform. Hierbei soll insbesondere die Ausrichtung eines Deckelteils und eines Bodenteils aneinander möglichst einfach handhabbar gestaltet werden. Erfindungsgemäß sieht die Gießform hierzu Zentrierelemente vor, welche eine gewünschte Anordnung zwischen einem Deckelteil und einem Bodenteil der Gießform erreichen. Die erfindungsgemäßen Zentrierelemente können weiterhin genutzt werden, um die beiden Teile, d. h. das Deckelteil und das Bodenteil, zusammenzuhalten. Somit können in vorteilhafter Weise zwei Funktionen in einem gemeinsamen Strukturelement der erfindungsgemäßen Gießform miteinander vereint werden. Die erfindungsgemäßen Gießformen können weiterhin die Bildung von Graten an den Spacern möglichst vermeiden. Eine weitere Aufgabe der Erfindung ist die Vermeidung von Lufteinschlüssen in den hergestellten Spacern. Die Erfindung beschäftigt sich auch mit der Aufgabe, die Ausbildung eines Angusses möglichst zu vermeiden, oder zumindest den beim Abformen gebildeten Anguss möglichst klein und leicht entfernbar zu halten.

Zudem ist es eine Zielsetzung der Erfindung, die Entnahme des geformten Spacers aus der Gießform möglichst leicht, schnell und für den medizinischen Anwender sicher zu gestalten. Insbesondere soll die Gefahr von Schnittverletzungen vermieden werden, welche aufgrund von scharfkantigen Graten entstehen können. Eine weitere Gefahr von Schnittverletzungen kann dadurch entstehen, dass der medizinische Anwender beim Aufschneiden einer Gießform mit einem Skalpell abrutscht. In einigen Ausführungsformen ermöglicht die erfindungsgemäße Gießform daher ein einfaches, sicheres und verletzungsfreies Öffnen der Gießform. Eine weitere Aufgabe der Erfindung ist auch die möglichst kostengünstige Herstellung der Gießform. Dies betrifft beispielsweise die Auswahl der für die Gießform verwendeten Materialien, und eine möglichst einfache geometrische Ausgestaltung der Gießform.

Einzelne, mehrere oder alle diese Aufgaben werden durch die hierin beschriebenen Gießformen gelöst, insbesondere diejenigen, welche in den Ansprüchen beschrieben sind.

### BEVORZUGTE AUSFÜHRUNGSFORMEN

Ein Aspekt der Erfindung betrifft in einer ersten Ausführungsform eine Gießform zur Herstellung eines Acetabulum-Spacers, aufweisend ein Bodenteil mit einem Rand, der das Bodenteil nach außen begrenzt, einer gewölbten, kreisförmigen Mulde und einer Sicke, die umlaufend um die Mulde angeordnet ist, wobei die Sicke zur Aufnahme von überschüssigem Knochenzement ausgebildet und eingerichtet ist,
und ein Deckelteil mit einer Vielzahl von Zentrierelementen, welche ausgebildet und eingerichtet sind, in den Rand einzugreifen, und dadurch das Deckelteil zentriert an dem Bodenteil auszurichten.

Eine zweite Ausführungsform betrifft eine Gießform gemäß der ersten Ausführungsform, wobei das Deckelteil mindestens drei Zentrierelemente aufweist.

Eine dritte Ausführungsform betrifft eine Gießform nach einer der vorangehenden Ausführungsformen, wobei das Bodenteil weiterhin ein Rastelement aufweist, wobei die Zentrierelemente ausgebildet und eingerichtet sind, in das Rastelement einzugreifen.

Eine vierte Ausführungsform betrifft eine Gießform gemäß der dritten Ausführungsform, wobei das Rastelement als Rille oder Vorsprung ausgebildet ist, welche(r) am Rand umlaufend um das Bodenteil angeordnet ist.

Eine fünfte Ausführungsform betrifft eine Gießform nach einer der vorangehenden Ausführungsformen, wobei die Vielzahl von Zentrierelementen jeweils gleichmäßig zueinander an dem Deckelteil beabstandet sind.

Eine sechste Ausführungsform betrifft eine Gießform nach einer der vorangehenden Ausführungsformen, wobei die Zentrierelemente jeweils einen Haken zum Eingriff in den Rand, bevorzugt gegebenenfalls zum Eingriff in ein Rastelement, aufweisen.

Eine siebte Ausführungsform betrifft eine Gießform nach einer der vorangehenden Ausführungsformen, wobei das Deckelteil weiterhin eine Kante aufweist, die zum Abtrennen eines in der Gießform hergestellten Spacers ausgebildet und eingerichtet ist.

Eine achte Ausführungsform betrifft eine Gießform nach einer der vorangehenden Ausführungsformen, wobei das Deckelteil weiterhin eine Rinne aufweist, wobei die Rinne ausgebildet und angeordnet ist, um in einem assemblierten Zustand der Gießform gegenüberliegend zu einem Randbereich der Mulde angeordnet zu werden.

Eine neunte Ausführungsform betrifft eine Gießform nach einer der vorangehenden Ausführungsformen, weiterhin aufweisend mindestens eine Entlüftungsöffnung, wobei die mindestens eine Entlüftungsöffnung bevorzugt gegebenenfalls in der Rinne gemäß der achten Ausführungsform angeordnet ist.

Eine zehnte Ausführungsform betrifft eine Gießform gemäß der neunten Ausführungsform, wobei die mindestens eine Entlüftungsöffnung einen Durchmesser von weniger als 1,0 mm, bevorzugt weniger als 0,5 mm aufweist.

Eine elfte Ausführungsform betrifft eine Gießform gemäß einer der vorangehenden Ausführungsformen, wobei die Gießform weiterhin eine Durchführung aufweist, welche zur Verbindung der Mulde mit der Sicke in einem assemblierten Zustand der Gießform ausgebildet ist.

Eine zwölfte Ausführungsform betrifft eine Gießform nach einer der vorangehenden Ausführungsformen, wobei das Bodenteil und/oder das Deckelteil mindestens eine Grifflasche zum Öffnen der Gießform aufweist.

Eine dreizehnte Ausführungsform betrifft eine Gießform nach einer der vorangehenden Ausführungsformen, wobei das Bodenteil und/oder das Deckelteil eine Oberfläche mit einer Struktur aufweist, wobei die Struktur bevorzugt einen Steg, eine Rille oder eine Noppe umfasst.

Ein weiterer Aspekt betrifft die Verwendung einer Gießform nach einer der vorangehenden Ausführungsformen zur Herstellung eines Acetabulum-Spacers.

Ein weiterer Aspekt betrifft ein Verfahren zur Herstellung eines Acetabulum-Spacers, aufweisend die nachfolgenden Schritte,
Bereitstellen einer Gießform nach einer der Ausführungsformen 1 bis 13;
Befüllen der Gießform mit Knochenzement;
Inkontaktbringen des Bodenteils mit dem Deckelteil, wobei die Zentrierelemente in den Rand eingreifen, und dadurch das Deckelteil zentriert an dem Bodenteil ausrichten;
Härten des Knochenzements in der Gießform;
Dadurch Erhalt eines Acetabulum-Spacers.

### KURZE BESCHREIBUNG DER ZEICHNUNGEN

**Figur 1** zeigt beispielhaft eine erste Ausführungsform einer erfindungsgemäßen Gießform in einer Querschnittsansicht.
**Figur 2** zeigt beispielhaft eine erfindungsgemäße Gießform in einer Aufsicht.
**Figur 3** zeigt beispielhaft eine erfindungsgemäße Gießform in einer Seitenansicht.
**Figur 4** zeigt beispielhaft eine geöffnete erfindungsgemäße Gießform in einer Seitenansicht.

### AUSFÜHRLICHE BESCHREIBUNG

Zu den hierin beschriebenen Ausführungsformen, deren Elemente ein bestimmtes Merkmal (z.B. ein Material) "aufweisen", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "aufweisen" oder "aufweisend" verwendet.

Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist.

Soweit nicht anders angegeben oder aus dem Zusammenhang eindeutig ausgeschlossen, ist es grundsätzlich möglich und wird hiermit eindeutig in Betracht gezogen, dass Merkmale unterschiedlicher Ausführungsformen auch in den anderen hierin beschriebenen Ausführungsformen vorhanden sein können. Ebenso wird grundsätzlich erwogen, dass alle Merkmale, die hierin in Zusammenhang mit einem Verfahren beschrieben werden, auch für die hierin beschriebenen Gießformen und Spacern anwendbar sind, und umgekehrt. Lediglich aus Gründen der knapperen Darstellung werden alle diese erwogenen Kombinationen nicht in allen Fällen explizit aufgeführt.

Ein erster Aspekt der Erfindung betrifft eine Gießform zur Herstellung von Spacern. Die erfindungsgemäße Gießform ist insbesondere zur Herstellung eines Acetabulum-Spacers geeignet. Ein solcher Spacer wird hierin auch als Acetabulum-Cup-Spacer bezeichnet. Ein solcher Spacer bildet die Form der Gelenkpfanne eines Hüftgelenks nach. Acetabulum-Spacer weisen üblicherweise eine schalenförmige Form mit einer konkaven Fläche zur Aufnahme eines Gelenkkopfs oder Spacerkopfs auf. Weiterhin weist ein Acetabulum-Spacer üblicherweise eine konvexe Rückseite auf, die mit Knochenzement im Acetabulum angebracht werden kann.

Die erfindungsgemäße Gießform weist ein Bodenteil und ein Deckelteil auf. Das Bodenteil kann formschlüssig und/oder kraftschlüssig mit dem Deckelteil verbindbar sein. Das Bodenteil weist einen Rand auf, der das Bodenteil nach außen begrenzt. Das Bodenteil weist weiterhin eine gewölbte, kreisförmige Mulde auf. Das Bodenteil weist eine Sicke auf. Die Sicke ist umlaufend um die Mulde des Bodenteils angeordnet. Die Sicke ist zur Aufnahme von überschüssigem Knochenzement ausgebildet und eingerichtet.

Das Deckelteil weist eine Vielzahl von Zentrierelementen auf. Die Zentrierelemente sind ausgebildet und eingerichtet, in den Rand des Bodenteils einzugreifen, und dadurch das Deckelteil zentriert an dem Bodenteil auszurichten.

Eine Ausführungsform der Erfindung betrifft demnach eine Gießform zur Herstellung eines Acetabulum-Spacers, aufweisend ein Bodenteil mit einem Rand, der das Bodenteil nach außen begrenzt, einer gewölbten, kreisförmigen Mulde und einer Sicke, die umlaufend um die Mulde angeordnet ist, wobei die Sicke zur Aufnahme von überschüssigem Knochenzement ausgebildet und eingerichtet ist,
und ein Deckelteil mit einer Vielzahl von Zentrierelementen, welche ausgebildet und eingerichtet sind, in den Rand einzugreifen, und dadurch das Deckelteil zentriert an dem Bodenteil auszurichten.

Die Gießform ist bevorzugt zweiteilig ausgebildet. Hierzu kann das Bodenteil lösbar mit dem Deckelteil verbindbar sein. Bevorzugt können die Zentrierelemente ausgebildet und eingerichtet sein, eine lösbare Verbindung des Deckelteils mit dem Bodenteil herzustellen.

Die Gießform kann eine im wesentlichen kreisförmige Grundform aufweisen. Hierbei kann der Rand des Bodenteils die Begrenzung der im wesentlichen kreisförmigen Grundform der Gießform bilden.

Die Mulde des Bodenteils kann eine konkave Form aufweisen. Das Deckelteil kann eine konvexe Form aufweisen.

Die Gießform kann einen Hohlraum aufweisen, welcher durch die Mulde des Bodenteils und einen Teil des Deckelteils ausgebildet wird. Ein solcher Hohlraum kann die Form des in der Gießform herzustellenden Spacers definieren. Erfindungsgemäß sind in diesem Zusammenhang Spacer bevorzugt, welche eine schalenförmige Form aufweisen. Daher kann der Hohlraum eine schalenförmige Form aufweisen. Der Hohlraum kann eine glatte Oberfläche aufweisen. In einer Ausführungsform weisen sowohl das Deckelteil als auch das Bodenteil eine vollständig glatte innere Oberfläche auf. In einigen Ausführungsformen ist die Gießform ausgebildet, Spacer mit Oberflächenstrukturen herzustellen. Hierzu kann eine innere Oberfläche des Deckelteils und/oder eine innere Oberfläche des Bodenteils entsprechende Strukturen aufweisen. Beispiele für solche Strukturen umfassen Stege, Noppen und Vertiefungen. Eine solche strukturierte Oberfläche kann beispielsweise die Befestigung eines Acetabulum-Spacers in einem Hüftgelenk des Patienten erleichtern. Daher kann es vorteilhaft sein, wenn insbesondere das Deckelteil eine innere Oberfläche mit einer solchen Struktur aufweist.

Die Gießform kann einen Kunststoff aufweisen. Die Gießform kann vollständig oder teilweise aus Kunststoff bestehen. Dies ermöglicht eine kostengünstige Herstellung, beispielsweise durch ein Spritzgussverfahren. Beispielsweise können das Deckelteil und das Bodenteil jeweils getrennt voneinander im Spritzgussverfahren aus Kunststoff herstellbar sein. In einigen Ausführungsformen ist der Kunststoff ein thermoplastischer Kunststoff. Beispiele für verwendbare Kunststoffe umfassen Polyethylen und Polypropylen. Besonders bevorzugt sind Kunststoffe mit geringer Dichte wie z.B. LD-PE. Bevorzugt weist der Kunststoff eine Dichte von weniger als 0,94 g/cm³ bei 1 bar und 25 °C auf.

Erfindungsgemäß weist die Gießform eine Vielzahl von Zentrierelementen auf. Die Zentrierelemente sind ausgebildet und eingerichtet, in den Rand einzugreifen, und dadurch das Deckelteil zentriert an dem Bodenteil auszurichten. Die Zentrierelemente können eine gewinkelte Form aufweisen. Hierbei kann ein Teilbereich der Zentrierelemente ausgebildet sein, sich in einem assemblierten Zustand der Gießform in Richtung des Bodenteils zu erstrecken. Die Zentrierelemente können eine Lasche aufweisen. Dies kann es einem Benutzer der Gießform erleichtern, die Zentrierelemente mit der Hand zu führen. Dies kann den Zusammenbau oder das Öffnen der Gießform erleichtern.

In einer Ausführungsform weist das Deckelteil mindestens drei Zentrierelemente auf. Hierdurch kann eine definierte und sichere Ausrichtung von Deckelteil und Bodenteil erreicht werden. In einigen Ausführungsformen weist das Deckelteil drei, vier, fünf oder sechs Zentrierelemente auf. In einigen Ausführungsformen weist das Deckelteil mehr als sechs Zentrierelemente auf. Die Zentrierelemente können eine jeweils gleichartige Form aufweisen. Die Zentrierelemente können jeweils gleichmäßig zueinander an dem Deckelteil beabstandet sein. Beispielsweise können die Zentrierelemente an einer im wesentlichen kreisförmigen Gießform in einem Winkel von jeweils 120° oder jeweils 90° zueinander beabstandet sein. Insbesondere kann die Gießform drei Zentrierelemente aufweisen, die in einem Winkel von jeweils 120° zueinander angeordnet sind. Die Gießform kann in einer Ausführungsform vier Zentrierelemente aufweisen, die jeweils im Winkel von 90° zueinander angeordnet sind.

Die Zentrierelemente sind ausgebildet und eingerichtet, in den Rand der Gießform einzugreifen. Hierzu kann der Rand der Gießform ein Rastelement aufweisen, wobei die Zentrierelemente ausgebildet und eingerichtet sind, in das Rastelement einzugreifen. Beispielsweise kann das Rastelement als umlaufende Rille oder Vorsprung ausgebildet sein. Das Rastelement kann am Rand umlaufend um das Bodenteil angeordnet sein. Die Zentrierelemente können beispielsweise einen Haken aufweisen, welcher zum Eingriff in das Rastelement ausgebildet ist. Ein solcher Haken kann beispielsweise als Vorsprung an einer Lasche eines Zentrierelements ausgebildet sein.

Hierdurch kann eine feste Positionierung des Deckelteils relativ zu dem Bodenteil erreicht werden. Gleichzeitig kann die Gießform durch den Eingriff der Zentrierelemente in den Rand des Bodenteils zusammengehalten werden. Hierdurch kann ein Hohlraum der Gießform während der Herstellung eines Spacers formstabil gehalten werden.

Um die Gießform leichter öffnen zu können, kann die Gießform weiterhin Grifflaschen aufweisen. Bevorzugt handelt es sich bei den Grifflaschen um Elemente, welche sich von den hierin beschriebenen Zentrierelementen unterscheiden. Die Grifflaschen können am Deckelteil angeordnet sein. Die Grifflaschen können am Bodenteil angeordnet sein. In einigen Ausführungsformen weisen sowohl das Deckelteil als auch das Bodenteil Grifflaschen auf. Die Grifflaschen können das Öffnen der Gießform erleichtern, indem sie es einem Benutzer der Gießform erlauben, die Grifflaschen mit der Hand zugreifen und dadurch das Bodenteil und das Deckelteil auseinanderzuziehen und somit voneinander zu trennen. Hierdurch kann ein Benutzer der Vorrichtung einen in der Gießform hergestellten Spacer leichter entnehmen.

Üblicherweise muss ein Überschuss von Knochenzement in eine Gießform für Spacer gefüllt werden, um eine vollständige Befüllung der Gießform sicherzustellen. Bei der Herstellung eines Spacers mithilfe einer Gießform kann es daher vorkommen, dass ein in einen Hohlraum der Gießform eingefüllter Knochenzement beim Schließen der Gießform teilweise herausgedrückt wird. Daher weist die erfindungsgemäße Gießform eine Sicke auf, welche umlaufend um die Mulde angeordnet ist. Die Sicke ist zur Aufnahme von überschüssigem Knochenzement ausgebildet und eingerichtet. Somit kann Knochenzement, der beim Schließen der Gießform aus der Mulde gedrückt wird, in der Sicke aufgenommen werden. Hierdurch kann ein unerwünschter Kontakt eines Benutzers der Gießform mit Knochenzement vermieden werden. Weiterhin kann hierdurch Knochenzement bei einer Benutzung der Gießform sauber verarbeitet werden. In einer Ausführungsform weist die Gießform mehrere Sicken auf. Diese Sicken können benachbarter zueinander angeordnet sein.

Ein aus der Mulde herausgedrückter Knochenzement kann einen Anguss des Spacers ausbilden, welcher üblicherweise unerwünscht ist. Ein in der Sicke aufgenommener Knochenzement soll daher möglichst sauber von dem in der Gießform hergestellten Spacer abtrennbar sein. Hierzu weist die Gießform in einigen Ausführungsformen weiterhin eine Kante auf, die zum Abtrennen eines in der Gießform hergestellten Spacers ausgebildet und eingerichtet ist. Dies bedeutet, dass ein Knochenzement in der Mulde mithilfe der Kante von einem in der Sicke aufgenommenen Knochenzement getrennt werden kann.

Die Kante kann an dem Deckelteil der Gießform angeordnet sein. Die Kante kann bevorzugt derart an dem Deckelteil angeordnet sein, dass sie in einem assemblierten Zustand der Gießform mit einem Randbereich der Mulde abschließt. Beispielsweise kann die Kante an der nachfolgend beschriebenen Rinne des Deckelteils angeordnet sein.

Weiterhin kann alternativ oder zusätzlich eine Kante an dem Bodenteil der Gießvorrichtung angeordnet sein. Bevorzugt kann die Kante an einem Randbereich der Mulde angeordnet sein. In einer Ausführungsform ist eine Kante zwischen der Mulde und der Sicke des Bodenteils angeordnet.

Eine Kante des Deckelteils kann mit einer Kante des Bodenteils zusammenwirken, sodass die beiden Kanten sich nach Art einer Schere gegeneinander bewegen und gemeinsam die Abtrennung eines Angusses bewirken können.

Das Deckelteil der Gießform kann eine Rinne aufweisen. Diese Rinne kann bevorzugt einen Randbereich eines Hohlraums der Gießform ausbilden. Die Rinne kann ausgebildet sein, einen kreisförmigen Rand eines in der Gießform herstellbaren Spacers zu definieren. Die Rinne ist bevorzugt ausgebildet und angeordnet, in einem assemblierten Zustand der Gießform gegenüberliegend zu einem Randbereich der Mulde angeordnet zu werden.

Ein "assemblierter Zustand" der Gießform beschreibt hierin eine Konfiguration, bei dem das Deckelteil mit dem Bodenteil fest verbunden ist, wobei sich die Zentrierelemente des Deckelteils mit dem Rand des Bodenteils, bevorzugt mit einem Rastelement des Bodenteils, im Eingriff befinden. In einem solchen "assemblierten Zustand" weist die Gießform bevorzugt einen Hohlraum auf, welcher der Form eines in der Gießform herzustellenden Spacers entspricht. Die Gießform kann weiterhin mindestens eine Entlüftungsöffnung aufweisen. Die mindestens eine Entlüftungsöffnung kann bevorzugt in der oben beschriebenen Rinne des Deckelteils angeordnet sein. Eine solche Entlüftungsöffnung kann es ermöglichen, einen Hohlraum der Gießform vollständig oder nahezu vollständig mit Knochenzement zu befüllen. Hierdurch können Luftblaseneinschlüsse bei einem in der Gießform herstellbaren Spacer vermieden werden. Die Anordnung von Entlüftungsöffnungen an der Rinne des Deckelteils kann vorteilhaft sein, um Luft am höchsten Punkt eines Hohlraums der Gießform nach außen zu leiten. Es können mehrere Entlüftungsöffnungen an der Gießform angeordnet sein. Es können mehrere Entlüftungsöffnungen in regelmäßigen Abständen umlaufend an der Rinne des Deckelteils angeordnet sein. Die Entlüftungsöffnung oder die Entlüftungsöffnungen weisen bevorzugt einen Durchmesser von weniger als 1,0 mm auf. In einer Ausführungsform weisen die Entlüftungsöffnungen einen Durchmesser von weniger als 0,5 mm auf. Durch diese Dimensionierung der Entlüftungsöffnungen kann vermieden werden, dass beim Schließen der Gießform zu viel Knochenzement aus der Gießform gedrückt wird. Somit kann die Ausbildung von größeren Angüssen an einem in der Gießform hergestellten Spacer vermieden werden. An den kleinen Entlüftungsöffnungen entstehen keine oder nur geringfügige Angüsse, welche gegebenenfalls leicht entfernbar sind.

In einer Ausführungsform weist die Gießform weiterhin eine Durchführung auf, welche zur Verbindung der Mulde mit der Sicke in einem assemblierten Zustand der Gießform ausgebildet ist. Dies bedeutet, dass Knochenzement aus der Mulde in die Sicke entweichen kann, wenn die Gießform durch Zusammenfügen des Deckelteils mit dem Bodenteil in einen assemblierten Zustand gebracht wird. Die Durchführung kann zweckmäßig als Aussparung oder Bohrung, bevorzugt an einem oberen Randbereich der Mulde, ausgestaltet sein. Die Durchführung ist bevorzugt so ausgebildet, dass sie die Aufnahme von Knochenzement aus der Mulde in die Sicke in einem assemblierten Zustand der Gießform erlaubt.

Die Gießform kann eine Standfläche zum Aufstellen der Gießform auf einer Unterlage aufweisen. In einer Ausführungsform ist die Standfläche am Bodenteil der Gießform angeordnet. In einer Ausführungsform ist die Standfläche an dem Deckelteil der Gießform angeordnet. Die Standfläche kann die Form eines Hohlzylinders aufweisen.

Mit "Knochenzement" wird hierin sowohl ein "Knochenzementteig" bezeichnet, d.h. eine formbare Masse, die zu einem festen, nicht mehr verformbaren Material aushärtbar ist, als auch der entsprechende ausgehärtete Knochenzement bezeichnet.

In entsprechender Weise bezieht sich der Begriff "Anguss" hierin auf verformbaren oder ausgehärteten Knochenzement, der eine üblicherweise unerwünschte Struktur an einem in der Gießform hergestellten Spacer bildet. Ein solcher Anguss kann sich an einer Öffnung der Gießform ausbilden, z.B. an einer hierin beschriebenen Entlüftungsöffnung oder Durchführung. Insbesondere bei herkömmlichen Gießformen kann sich ein Anguss auch im Bereich von Trennungen einer mehrteiligen Gießform bilden.

Handelsübliche Knochenzemente werden in vielen Fällen als Kit angeboten, welches eine flüssige und eine feste Komponente enthält. Durch Mischen der flüssigen mit der festen Komponente kann der Anwender einen Knochenzementteig herstellen, der für eine begrenzte Zeit leicht verformbar ist. Bereits kurze Zeit nach dem Mischen wird der Knochenzementteig klebfrei, d. h. er haftet bei leichter Berührung mit einem Handschuh nicht mehr an diesem an (wie definiert in ISO 5833:2002). In diesem Zustand kann ein Knochenzementteig bevorzugt in eine erfindungsgemäße Gießform zur Herstellung von Spacern eingefüllt werden, beispielsweise mithilfe eines Rührspatels, oder bevorzugt mithilfe einer geeigneten Austragsvorrichtung. Handelsübliche Knochenzemente werden häufig auf Basis von Polymethylmethacrylat (PMMA) angeboten. Die erfindungsgemäße Gießform kann bevorzugt mit einem solchen PMMA-Knochenzement verwendet werden. Es ist jedoch grundsätzlich auch möglich, andere Arten von Knochenzementen zu verwenden.

Die Erfindung betrifft weiterhin die Verwendung einer hierin beschriebenen Gießform zur Herstellung eines Acetabulum-Spacers. Hierzu kann die Gießform wie hierin beschrieben mit Knochenzement befüllt werden, wobei der Knochenzement in der Gießform einer Härtung unterzogen werden kann, um einen festen Acetabulum-Spacer herzustellen. Die Gießform kann anschließend wie hierin beschrieben geöffnet werden, und der hergestellte Acetabulum-Spacer kann aus der Gießform entnommen werden.

Die Erfindung betrifft weiterhin ein Verfahren zur Herstellung eines Acetabulum-Spacers. Hierzu kann eine hierin beschriebene Gießform verwendet werden. Das Verfahren kann die folgenden Schritte aufweisen:
Bereitstellen einer hierin beschriebenen Gießform;
Befüllen der Gießform mit Knochenzement;
Inkontaktbringen des Bodenteils mit dem Deckelteil, wobei die Zentrierelemente in den Rand eingreifen, und dadurch das Deckelteil zentriert an dem Bodenteil ausrichten;
Härten des Knochenzements in der Gießform; und
Dadurch Erhalt eines Acetabulum-Spacers.

Das Verfahren kann weiterhin einen Schritt umfassen, in welchem Knochenzement aus einer Mulde der Gießform in eine Sicke der Gießform gedrückt wird.

Das Verfahren kann weiterhin das Entweichenlassen von Luft aus der Gießform durch eine Entlüftungsöffnung umfassen.

Das Verfahren kann weiterhin das Abtrennen eines in der Gießform hergestellten Spacers mithilfe einer Kante der Gießform umfassen.

Die oben dargestellten Schritte des Verfahrens können grundsätzlich in jeder zweckmäßigen Reihenfolge erfolgen. In einer Ausführungsform erfolgt die Durchführung des Verfahrens in der oben dargestellten Reihenfolge.

Ein weiterer Aspekt der Erfindung ist ein Spacer, der mithilfe einer hierin beschriebenen Gießform herstellbar ist, oder hergestellt ist.

### BEISPIELE

Die Erfindung wird nachfolgend anhand von Beispielen weiter verdeutlicht, die jedoch nicht als einschränkend zu verstehen sind. Dem Fachmann wird ersichtlich sein, dass anstelle der hier beschriebenen Merkmale andere äquivalente Mittel in ähnlicher Weise verwendet werden können.

Eine erfindungsgemäße Gießform kann beispielsweise wie folgt verwendet werden. Zur Herstellung eines Spacers in der Gießform kann zunächst ein Polymethylmethacrylat-Knochenzementteig in einer Schale oder anderen geeigneten Mischvorrichtung aus einer flüssigen Komponente und einer Pulverkomponente zubereitet werden. Das Vermischen des Zementkomponenten kann im einfachsten Fall mit einer Mischschüssel und mit einem Spatel vorgenommen werden. Danach kann der Knochenzementteig in die konkave Kavität des Bodenteils der Gießvorrichtung eingebracht werden. Dies kann zweckmäßig durch Einstreichen des Knochenzements mithilfe eines Spatels erfolgen. Anschließend kann das Deckelteil der Gießform auf das Bodenteil der Gießform gesetzt werden. Hierbei greifen die Zentrierelemente des Deckelteils in den Rand des Bodenteils ein. Hierdurch wird das Deckelteil im Verhältnis zum Bodenteil in einer gewünschten Position ausgerichtet, um die Herstellung eines Spacers zu ermöglichen. Beim Zusammenfügen des Deckelteils mit dem Bodenteil drückt eine konvexe Oberfläche des Deckelteils gegen den Knochenzement, der sich in der Mulde des Bodenteils befindet. Aufgrund dessen kann Knochenzement aus der Mulde des Bodenteils in eine Sicke gelangen, welche sich benachbart zu der Mulde des Bodenteils befindet. In der nunmehr assemblierten Gießform härtet der Knochenzement durch radikalische Polymerisation aus. Hierdurch bildet sich ein Acetabulum-Spacer. Durch Lösen der Zentrierelemente und Auseinanderziehen des Deckelteils vom Bodenteil mithilfe von Grifflaschen kann die Gießform geöffnet werden, um den hergestellten Spacer in der Gießform zur Entnahme freizulegen.

### FIGUREN

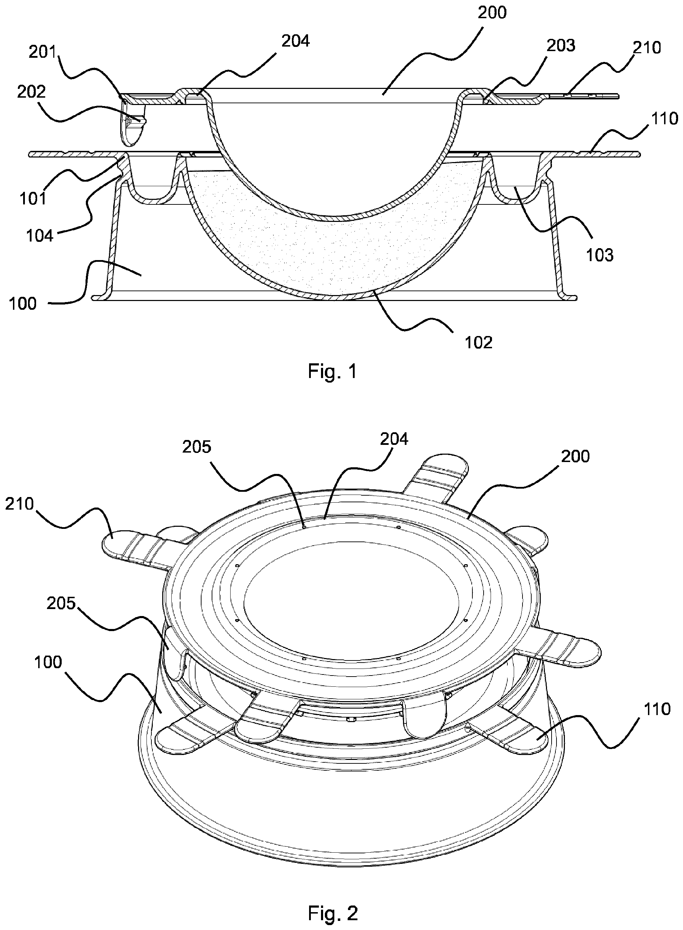

**Figur 1** zeigt beispielhaft eine erfindungsgemäße Gießform in einer Querschnittsansicht. Die Gießform weist ein Bodenteil 100 mit einem umlaufenden Rand 101 auf. Eine Mulde 102 zur Aufnahme von Knochenzement ist zentral in dem Bodenteil 100 angeordnet. Zwischen der Mulde 102 und dem Rand 101 ist eine Sicke 103 angeordnet. Die Sicke 103 ist zur Aufnahme von überschüssigem Knochenzement aus der Mulde 102 ausgebildet. Am Rand 101 des Bodenteils ist ein Rastelement 104 in Form einer umlaufenden Rille angeordnet. Weiterhin weist das Bodenteil 100 eine Vielzahl von Grifflaschen 110 auf. Das hier dargestellte Bodenteil 100 ist so ausgestaltet, dass es zur Benutzung auf einer ebenen Unterlage abgestellt werden kann. Die Gießform weist weiterhin ein Deckelteil 200 auf. An dem Deckelteil 200 sind mehrere Zentrierelemente 201 angeordnet, die laschenförmig ausgestaltet sind. Die Zentrierelemente 201 weisen hier Haken 202 auf, welche in Form von Vorsprüngen ausgebildet sind. Die Zentrierelemente 201 sind zum Eingriff in die Rille 104 am Rand 101 des Bodenteils 100 ausgebildet. Dies ermöglicht es, die Gießform in einem assemblierten Zustand zu halten, in welchem das Bodenteil 100 und des Deckelteil 200 in einer definierten Position zueinander angeordnet sind. Das Deckelteil 200 weist weiterhin eine Kante 203 auf, um in die Gießform eingefüllten Knochenzement am Randbereich 106 der Mulde 102 abzutrennen. Hierdurch kann vermieden werden, dass überschüssiger Knochenzement, welcher beim Zusammenbau von Bodenteil 100 und Deckelteil 200 in die Sicke 103 gelangt, an dem in der Gießform hergestellten Spacer haften bleibt. Das Deckelteil 200 weist weiterhin eine Rinne 204 auf, welche so ausgebildet und angeordnet ist, dass sie in einem assemblierten Zustand der Gießform, d. h., wenn das Deckelteil 200 fest mit dem Bodenteil 100 verbunden ist, gegenüberliegend zu einem Randbereich 104 der Mulde 106 angeordnet werden kann.

**Figur 2** zeigt beispielhaft eine erfindungsgemäße Gießform in einer Aufsicht. Das Deckelteil 200 weist mehrere Grifflaschen 210 auf, welche jeweils gleichmäßig zueinander beabstandet sind. Im hier gezeigten Beispiel sind vier Grifflaschen 210 kreisförmig in einem Winkel von je 90° zueinander entlang eines Randes des Deckelteils 200 angeordnet. Die Grifflaschen 210 erstrecken sich jeweils vom Rand des Deckelteils 200 seitlich nach außen. Das Deckelteil 200 weist weiterhin mehrere Entlüftungsöffnungen 205 auf, welche mit der Rinne 204 verbunden sind. Hierdurch kann Luft aus der Gießform am höchsten Punkt der Gießform nach außen abgegeben werden, wenn die Gießform zusammengebaut wird. Die Zentrierelemente 201 sind gewinkelt an dem Deckelteil 200 angeordnet, und weisen in Richtung des Randes 101 des Bodenteils 100, um beim Zusammenfügen des Deckelteils 200 mit dem Bodenteil 100 dort einzugreifen.

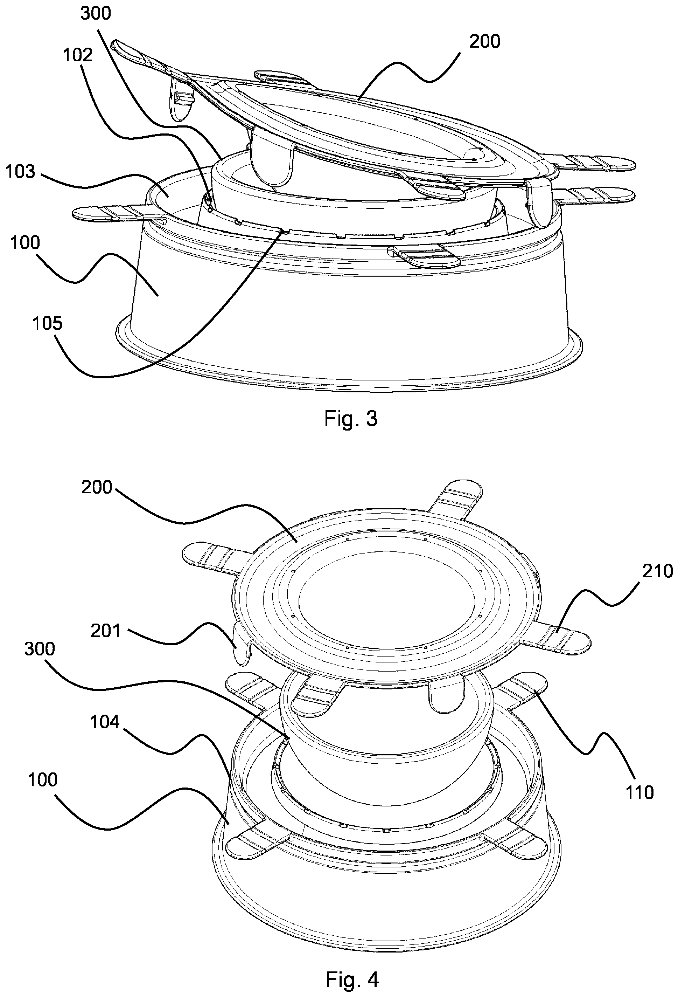

**Figur 3** zeigt beispielhaft eine erfindungsgemäße Gießform in einer Seitenansicht. In der Gießform befindet sich ein Spacer 300, welcher aus Knochenzement in der Mulde 102 der Gießform hergestellt ist. Zwischen der Mulde 102 und der Sicke 103 sind Durchführungen 105 angeordnet, welche die Abgabe von überschüssigem Knochenzement aus der Mulde 102 in die Sicke 103 erlauben, wenn die Gießform durch Verbinden des Bodenteils 100 mit dem Deckelteil 200 zusammengebaut wird. Ein solcher zusammengebauter Zustand wird hierin auch als "assemblierte" Gießform bezeichnet. In diesem assemblierten Zustand befinden sich die Zentrierelemente 201 im Eingriff mit dem Rand 101 des Bodenteils 100, und sind gegebenenfalls in einem Rastelement 104 verrastet. Nach der Herstellung des Spacers in der Gießform kann die Gießform geöffnet werden, indem die Grifflaschen 110, 210 auseinandergezogen werden, um das Bodenteil 100 von dem Deckelteil 200 zu trennen.

Gegebenenfalls können hierbei weiterhin die Zentrierelemente 201 gelöst und seitlich gebogen werden, um den Eingriff der Zentrierelemente 201 aus dem Rand 101 und gegebenenfalls dem Rastelement 104 zu lösen.

**Figur 4** zeigt beispielhaft eine geöffnete erfindungsgemäße Gießform in einer Seitenansicht. Das Bodenteil 100 und das Deckelteil 200 sind nach der Herstellung eines Spacers 300 in der Gießform unter Verwendung der Grifflaschen 110, 210 voneinander getrennt, um den Spacer 300 aus der Gießform zu entnehmen. Die Zentrierelemente 201 befinden sich nicht mehr im Eingriff mit dem Rastelement 104, welches als umlaufende Rille in dem Bodenteil 100 ausgestaltet ist. Deutlich erkennbar ist die Gießform als zweiteilige Vorrichtung ausgestaltet, welche als voneinander getrennte Teile ein Bodenteil 100 und ein Deckelteil 200 aufweist, welche lösbar miteinander in Eingriff gebracht und miteinander verbunden werden können.

### LISTE DER BEZUGSZEICHEN

- 100: Bodenteil
- 101: Rand
- 102: Mulde
- 103: Sicke
- 104: Rastelement
- 105: Durchführung
- 106: Randbereich der Mulde 102
- 110: Grifflasche
- 200: Deckelteil
- 201: Zentrierelement
- 202: Haken
- 203: Kante
- 204: Rinne
- 205: Entlüftungsöffnung
- 210: Grifflasche

## Patentansprüche

1. Gießform zur Herstellung eines Acetabulum-Spacers, aufweisend ein Bodenteil (100) mit einem Rand (101), der das Bodenteil (100) nach außen begrenzt, einer gewölbten, kreisförmigen Mulde (102) und einer Sicke (103), die umlaufend um die Mulde (102) angeordnet ist, wobei die Sicke (103) zur Aufnahme von überschüssigem Knochenzement ausgebildet und eingerichtet ist,
und ein Deckelteil (200) mit einer Vielzahl von Zentrierelementen (201), welche ausgebildet und eingerichtet sind, in den Rand (101) einzugreifen, und dadurch das Deckelteil (200) zentriert an dem Bodenteil (100) auszurichten.

2. Gießform nach Anspruch 1, wobei das Deckelteil (200) mindestens drei Zentrierelemente (201) aufweist.

3. Gießform nach einem der vorangehenden Ansprüche, wobei das Bodenteil (100) weiterhin ein Rastelement (104) aufweist, wobei die Zentrierelemente (201) ausgebildet und eingerichtet sind, in das Rastelement (104) einzugreifen.

4. Gießform nach Anspruch 3, wobei das Rastelement (104) als Rille oder Vorsprung ausgebildet ist, welche(r) am Rand (101) umlaufend um das Bodenteil (100) angeordnet ist.

5. Gießform nach einem der vorangehenden Ansprüche, wobei die Vielzahl von Zentrierelementen (201) jeweils gleichmäßig zueinander an dem Deckelteil (200) beabstandet sind.

6. Gießform nach einem der vorangehenden Ansprüche, wobei die Zentrierelemente (201) jeweils einen Haken (202) zum Eingriff in den Rand (101), bevorzugt gegebenenfalls zum Eingriff in ein Rastelement (104), aufweisen.

7. Gießform nach einem der vorangehenden Ansprüche, wobei das Deckelteil (200) weiterhin eine Kante (203) aufweist, die zum Abtrennen eines in der Gießform hergestellten Spacers ausgebildet und eingerichtet ist.

8. Gießform nach einem der vorangehenden Ansprüche, wobei das Deckelteil (200) weiterhin eine Rinne (204) aufweist, wobei die Rinne (204) ausgebildet und angeordnet ist, um in einem assemblierten Zustand der Gießform gegenüberliegend zu einem Randbereich (106) der Mulde (102) angeordnet zu werden.

9. Gießform nach einem der vorangehenden Ansprüche, weiterhin aufweisend mindestens eine Entlüftungsöffnung (205), wobei die mindestens eine Entlüftungsöffnung bevorzugt gegebenenfalls in der Rinne (204) nach Anspruch 8 angeordnet ist.

10. Gießform nach Anspruch 9, wobei die mindestens eine Entlüftungsöffnung einen Durchmesser von weniger als 1,0 mm, bevorzugt weniger als 0,5 mm aufweist.

11. Gießform nach einem der vorangehenden Ansprüche, wobei die Gießform weiterhin eine Durchführung (105) aufweist, welche zur Verbindung der Mulde (102) mit der Sicke (103) in einem assemblierten Zustand der Gießform ausgebildet ist.

12. Gießform nach einem der vorangehenden Ansprüche, wobei das Bodenteil (100) und/oder das Deckelteil (200) mindestens eine Grifflasche (110, 210) zum Öffnen der Gießform aufweist.

13. Gießform nach einem der vorangehenden Ansprüche, wobei das Bodenteil (100) und/oder das Deckelteil (200) eine Oberfläche mit einer Struktur aufweist, wobei die Struktur bevorzugt einen Steg, eine Rille oder eine Noppe umfasst.

14. Verwendung einer Gießform nach einem der vorangehenden Ansprüche zur Herstellung eines Acetabulum-Spacers.

15. Verfahren zur Herstellung eines Acetabulum-Spacers, aufweisend die nachfolgenden Schritte,
Bereitstellen einer Gießform nach einem der Ansprüche 1 bis 13;
Befüllen der Gießform mit Knochenzement;
Inkontaktbringen des Bodenteils (100) mit dem Deckelteil (200), wobei die Zentrierelemente (201) in den Rand (101) eingreifen, und dadurch das Deckelteil (200) zentriert an dem Bodenteil (100) ausrichten;
Härten des Knochenzements in der Gießform;
Dadurch Erhalt eines Acetabulum-Spacers.
